Europäisches Patentamt

(19) European Patent Office

Office européen des brevets

(11) Veröffentlichungsnummer: **0 127 160**
**B1**

(12) # EUROPÄISCHE PATENTSCHRIFT

(45) Veröffentlichungstag der Patentschrift:
**01.04.87**

(51) Int. Cl.⁴: **A 61 K 31/70,** A 61 K 7/48,
A 61 K 9/06, A 61 K 9/14

(21) Anmeldenummer: **84105992.6**

(22) Anmeldetag: **25.05.84**

(54) **Äusserlich anzuwendende Arzneimittelzubereitung enthaltend Adenosin zur Behandlung von Herpes.**

(30) Priorität: **27.05.83 DE 3319282**

(43) Veröffentlichungstag der Anmeldung:
**05.12.84 Patentblatt 84/49**

(45) Bekanntmachung des Hinweises auf die Patenterteilung:
**01.04.87 Patentblatt 87/14**

(84) Benannte Vertragsstaaten:
**AT BE CH DE FR GB IT LI LU NL SE**

(56) Entgegenhaltungen:
**DE - A - 2 330 902
FR - A - 1 440 795
FR - A - 2 358 155
US - A - 4 044 122**

**Die Akte enthält technische Angaben, die nach dem Eingang der Anmeldung eingereicht wurden und die nicht in dieser Patentschrift enthalten sind.**

(73) Patentinhaber: **GÖDECKE AKTIENGESELLSCHAFT,
Salzufer 16, D-1000 Berlin 10 (DE)**

(72) Erfinder: **Osswald, Hartmut, Prof. Dr. Med., Kieferweg 1,
D-7808 Waldkirch/Kollnau (DE)**

## Beschreibung

Es wurde gefunden, dass Adenosin bei äuserlicher Anwendung überraschend eine ausgezeichnete Wirkung bei der Bekämpfung der verschiedenen Herpesarten, insbesondere von Herpes zoster, Herpes labialis und Herpes genitalis besitzt.

Adenosin ist ein seit langer Zeit bekanntes Nukleosid, das in Form seiner Phosphate im Stoffwechsel eine bedeutende Rolle spielt.

Als Arzneimittel spielt Adenosin jedoch bisher unter anderem deshalb keine Rolle, weil es bei enteraler und parenteraler Gabe extrem rasch metabolisiert wird. Eine äusserliche Anwendung von Adenosin ist bisher überhaupt noch nicht bekannt geworden.

Erfindungsgemäss wird Adenosin in einer Konzentration von 5–30%, vorzugsweise etwa 10–20% in einen neutralen Träger eingearbeitet und 3–10mal täglich dünn auf befallene Hautstellen aufgetragen. Charakteristischerweise ist ein Erfolg der Behandlung an der Abnahme der Schmerzhaftigkeit und Schmerzempfindlichkeit bei Berührung der erkrankten Stellen schon innerhalb der ersten 12 Stunden nach Beginn der Behandlung festzustellen. In vielen Fällen verschwinden Schmerzen bereits innerhalb des ersten Behandlungstages völlig. Als neutrale Träger kommen alle üblichen für Salben, Cremes, Lotions, Sprays, Puder oder ähnliche Zubereitungen verwendeten Grundstoffe in Frage. Hierzu gehören z.B. Alkohol, Propylenglykol, Glycerol, Cetylstearylalkohol, Vaseline, Wollwachsalkohole, Wollfett, Hartfette, Talkum und gegebenenfalls Konservierungsmittel, wie PHB-Ester oder Sorbinsäure.

Vorzugsweise werden jedoch solche Zubereitungen eingesetzt, die dafür sorgen, dass die behandelten Hautpartien möglichst lange Zeit mit dem Wirkstoff in Berührung bleiben und daher ein gutes Haftungsvermögen auf der Hautoberfläche besitzen.

Es ist weiterhin für die Behandlung günstig, wenn die Hautpartien möglichst trocken gehalten werden, so dass sich unter diesem Gesichtspunkt Puder, wasserfreie Salben oder Lippenstifte und andere wasserfreie Zubereitungen besonders empfehlen. Der Wirkungsmechanismus der erfindungsgemässen Zubereitungen ist bisher noch nicht aufgeklärt. Es wird jedoch angenommen, dass Adenosin die Vermehrung der Herpesviren in den Wirtszellen hemmt.

Der Vorteil der äusserlich anzuwendenden Arzneimittelzubereitung liegt insbesondere darin, dass sehr hohe Wirkstoffkonzentrationen ausschliesslich an der erkranten Hautstelle errreicht werden. Bedingt durch den schnellen Metabolismus des Adenosins sind erfindungsgemäss systemische Wirkungen des Wirkstoffs nicht zu erwarten. Hierdurch unterscheidet sich die erfindungsgemässe Verwendung von derjenigen bekannter Virustatika, die z.T. erhebliche systemisch bedingte Nebenwirkungen aufweisen.

Gegenstand der Erfindung ist die Verwendung von Adenosin zur Herstellung eines Arzneimittels für die Bekämpfung von Herpes.

Die folgenden Beispiele dienen der näheren Erläuterung der Erfindung:

### Beispiel 1

20g Adenosin werden mit 80g ungt. alcohol. Lanae gemischt. Die erhaltene Salbe kann täglich mehrmals auf von Herpes befallene Haut- oder Schleimhautpartien dünn aufgetragen werden.

### Beispiel 2

10g Adenosin wird in einer Mischung von 17,5g Äthanol, und 62,5g gereinigtem Wasser unter leichtem Erwärmen gelöst. Zu dieser Lösung gibt man 10,0g Glycerol und filtriert die erhaltene Lösung über ein Membranfilter mit einem Porendurchmesser von 20 μm.

Man erhält eine klare Lösung, die aufgrund des Alkoholgehaltes steril bleibt. Zur Behandlung wird die befallene Hautstelle mehrmals täglich bepinselt.

### Beispiel 3

10g Adenosin wird in 60g warmem Wasser, dem gegebenenfalls ein Konservierungsmittel beigegeben wird, gelöst und zusammen mit 5g Zinkoxid, 5g Talkum und 20g Glycerol zu einer Lotio verarbeitet. Zinkoxid und Talkum werden vor Zugabe gut gemischt und gesiebt und dann in dünner Schicht in einem Trockenschrank während 1 Stunde auf 180° erhitzt.

Die erhaltene Lotio wird nach Aufschütteln mehrmals täglich dünn auf befallene Hautpartien aufgetragen.

### Beispiel 4

20g Adenosin werden in 80g einer wasserfreien Salbengrundlage eingearbeitet. Die Salbengrundlage wird wie folgt hergestellt:
Bestandteile: Aerosil[R] 8,0g
Isopropylmyristat
Isopropylpalmitat
Paraffin (dickflüssig)
ad 100,0g.

Die flüssigen Komponenten werden gemischt. In einem Teil davon wird Aerosil® zu einem klumpenfreien Gel verrieben; diesem Gel setzt man den Rest der Lösung porionenweise unter Rühren zu.

Nach Homogenisierung wird die erhaltene wasserfreie Salbe steril abgefüllt. Sie wird mehrmals täglich dünn auf befallene Hautstellen aufgetragen.

### Beispiel 5

15g fein vermahlenes Adenosin werden mit 85g einer Pudergrundlage, welche aus 48% Talkum, 50% Stärke (nichtquellend) und 2,0g Aerosil® besteht, trocken intensiv vermischt und durch ein feines Sieb (ISO-Nr. 1000) gegeben. Man erhält einen Puder mit einer Korngrösse von 50 μm, der mehrmals täglich dünn auf befallene Hautstellen aufgebracht wird.

Beispiel 6

80g Hartfett werden bei 40°C geschmolzen. In die Schmelze werden 10g Isopropylmyristat zusammen mit 10g Adenosin eingerührt. Die Gesamtschmelze wird dann in einer Kolloidmühle feinst vermahlen und bei einer Temperatur von 33 – 35°C als Cremeschmelze in zylindrische Formen gegossen. Die nach dem Erkalten erhaltenen Stifte werden in einen Applikator zur besseren Handhabung eingesteckt. Der erhaltene medizinische Lippenstift kann mehrmals täglich zur Behandlung befallener Hautstellen verwendet werden.

**Patentansprüche für die Vertragsstaaten BE, CH, DE, FR, GB, IT, LI, LU, NL, SE**

1. Verwendung von Adenosin zur Herstellung eines Arzneimittels für die Bekämpfung von Herpes.

2. Verwendung von Adenosin gemäss Anspruch 1, zur Herstellung eines Arzneimittels für die Bekämpfung von Herpes labialis.

3. Verwendung von Adenosin gemäss Anspruch 1, zur Herstellung eines Arzneimittels für die Bekämpfung von Herpes genitalis.

4. Verwendung von Adenosin gemäss Anspruch 1, zur Herstellung eines Arzneimittels für die Bekämpfung von Herpes zoster.

**Patentansprüche für den Vertragsstaat AT**

1. Verfahren zur Herstellung einer äusserlich zu verwendenden Zubereitung von Adenosin zur Bekämpfung von Herpes, dadurch gekennzeichnet, dass man Adenosin in an sich bekannter Weise in einer Konzentration von 5 – 30% in einen neutralen Arzneimittelträger einarbeitet.

2. Verfahren gemäss Anspruch 1, dadurch gekennzeichnet, dass der Arzneimittelträger eine wasserfreie Salbe oder ein wasserfreier Lippenstift ist.

3. Verfahren gemäss Anspruch 1, dadurch gekennzeichnet, dass der Arzneimittelträger ein Puder ist.

**Claims for the contracting states BE, CH, DE, FR, GB, IT, LI, LU, NL, SE**

1. Use of adenosine for the manufacture of a pharmaceutical preparation for the treatment of herpes.

2. Use of adenosine according to claim 1 for the manufacture of a pharmaceutical preparation for the treatment of herpes labialis.

3. Use of adenosine according to claim 1 for the manufacture of a pharmaceutical preparation for the treatment of herpes genitalis.

4. Use of adenosine according to claim 1 for the manufacture of a pharmaceutical preparation for the treatment of herpes zoster.

**Claims for the contracting state AT**

1. Process for the manufacture of a pharmaceutical preparation for topical application containing adenosine for the treatment of herpes, characterized by incorporating adenosine in a generally known manner into a neutral pharmaceutical carrier in a concentration of 5 to 30%

2. Process according to claim 1, wherein the pharmaceutical carrier is an anhydrous ointment or an anhydrous lipstick.

3. Process according to claim 1, wherein the pharmaceutical carrier is a powder.

**Revendications pour les Etats contractants BE, CH, DE, FR, GB, IT, LI, LU, NL, SE**

1. Utilisation d'adénosine pour la préparation d'un médicament pour la lutte contre l'herpès.

2. Utilisation d'adénosine selon la revendication 1, pour la préparation d'un médicament pour la lutte contre l'herpès labial.

3. Utilisation d'adénosine selon la revendication 1, pour la préparation d'un médicament pour la lutte contre l'herpès génital.

4. Utilisation d'adénosine selon la revendication 1, pour la préparation d'un médicament pour la lutte contre l'herpès zoster.

**Revendications pour l'Etat contractant AT**

1. Procédé de préparation d'une composition à usage externe à base d'adénosine pour la lutte contre l'herpès, caractérisé en ce que, d'une façon connue en soi, l'on incorpore de l'adénosine à une concentration de 5 à 30% dans un support médicamenteux neutre.

2. Procédé selon la revendication 1, caractérisé en ce que le support médicamenteux est un onguent anhydre ou un bâton à lèvre anhydre.

3. Procédé selon la revendication 1, caractérisé en ce que le support médicamenteux est une poudre.